Europäisches Patentamt

European Patent Office

Office européen des brevets

(11) Publication number: **0 034 459**
**B1**

# EUROPEAN PATENT SPECIFICATION

(45) Date of publication of patent specification: **18.12.85**

(21) Application number: **81300540.2**

(22) Date of filing: **10.02.81**

(51) Int. Cl.⁴: **G 01 N 27/22, G 01 N 33/10, G 01 N 9/36, G 01 G 13/08**

(54) Cereal grain moisture content measuring apparatus.

(30) Priority: **14.02.80 JP 17362/80**

(43) Date of publication of application:
**26.08.81 Bulletin 81/34**

(45) Publication of the grant of the patent:
**18.12.85 Bulletin 85/51**

(84) Designated Contracting States:
**BE CH DE GB IT LI NL**

(56) References cited:
**DE-C- 699 791**
**DE-C- 824 399**
**DE-C-1 121 378**
**US-A-3 691 457**
**US-A-4 050 016**

**Patents Abstracts of Japan, vol. 3, no. 136, 13.**
**November 1979, page 142E151**

(73) Proprietor: **SATAKE ENGINEERING CO., LTD.**
**19-10, Ueno 1-chome**
**Taito-ku Tokyo 110 (JP)**

(72) Inventor: **Satake, Toshihiko**
**2-38, Saijonishihonmachi**
**Higashihiroshima-shi (JP)**

(74) Representative: **Hayward, Denis Edward**
**Peter et al**
**Lloyd Wise, Tregear & Co. Norman House**
**105-109 Strand**
**London WC2R OAE (GB)**

Courier Press, Leamington Spa, England.

## Description

This invention relates to cereal grain moisture content measuring apparatus of the electrostatic capacity type.

Apparatus of the prior art for measuring the moisture content of cereal grains by electrostatic capacity include an electrostatic sensor built into the measuring chamber. Since the density of cereal grains in the measuring chamber may vary depending on the shape, surface coarseness and other conditions of the cereal grains, large errors have tended to occur in the values obtained in measuring the moisture content of cereal grains by means of the electrostatic sensor. Also, the apparatus of the prior art of the type described are complex in construction and mechanical and electrical failures have often occurred.

DE—C—1121378 describes an apparatus for measuring the moisture content of pulverulent and granular materials by a continuous flow process. The material is fed continuously from a hopper in a vertical stream through a vessel in the form of an upright open-ended tube with a convergent discharge cone at its lower end, and a signal representative of the moisture content of the material is generated continuously by a sensor including capacitor electrodes placed in the sides of the tube, while another signal representative of the weight of the tube and its contents is continuously generated by a tube-weighing device. The weight signal is then used to correct the moisture content reading. A problem with this apparatus is that it is difficult to accurately and correctly measure the weight of the material within the tubular flow vessel because the material to be measured is continuously impinging against the walls of the flow vessel which introduces a dynamic effect that prevents the true weight of the material from being sensed.

By contrast, it is an object of the present invention to achieve an improved cereal grain moisture content measuring apparatus of the electrostatic capacity type which can operate substantially continuously but does not suffer from the weight measurement being vitiated by dynamic effect as in the prior patent just discussed.

According to the present invention, there is provided apparatus for measuring moisture content of cereal grains, comprising a vessel for receiving successive samples of cereal grains each having a predetermined volume, said vessel having a substantially tubular body with an upper open end and a lower open end, feeding means for feeding cereal grains into said vessel, sensor means associated with said vessel for detecting electrostatic capacity of a sample of cereal grains in said vessel to generate a signal representative of the moisture content of the cereal grains, a measuring unit for measuring the weight of each sample of cereal grains having said predetermined volume to generate a signal representative of the measured weight, and a correction circuit electrically connected to said sensor means and said measuring unit for receiving the signal representative of the moisture content and the signal representative of the weight, operating to correct the moisture content signal in accordance with changes in density of the cereal grains, characterized in that said vessel has associated therewith valve means which is movable between a closed position in which it closes the lower open end of said vessel to retain a sample of cereal grains to be measured in said vessel, and an open position in which it opens the lower open end of said vessel to allow the measured cereal grain sample to be discharged out of said vessel, with actuating means for moving said valve means between the closed and open positions;

in that said feeding means comprises a feeding trough having an upstream end, and a downstream end located above the upper open end of said vessel, and vibrating means for vibrating said feeding trough, when said vibrating means is activated and said valve means is in its closed position, to cause cereal grains to be gradually fed into said vessel by the feeding trough until the thus fed cereal grains are overflowed from the upper open end of said vessel to form an angle of repose at the top of the fed cereal grains so that a sample of the cereal grains having the predetermined volume is received in said vessel, said feeding trough remaining stationary so that no cereal grains are fed when the vibrating means is deactivated; and

in that signal-generating means is electrically connected to said actuating means for said valve means and to said vibrating means of said feeding means for generating and supplying a first signal to said vibrating means to deactivate the same when a sample of the cereal grains having the predetermined volume has been fed into said vessel by said feeding trough, and for generating and supplying a second signal to said actuating means so as to cause the valve means to be moved into said open position when the detection by said sensor means and the measurement by said measuring unit have been completed.

By virtue of the aforesaid features, accurate determinations of the moisture contents of samples of cereal grains can be obtained by avoiding errors that might otherwise occur.

The apparatus has a structure which is so simple that mechanical and electrical failures are unlikely, and the performance of the apparatus in determining the moisture content of samples of cereal grains is such that grain moisture content determinations can be obtained with high accuracy at all times.

Arrangements embodying the invention will now be described by way of example with reference to the accompanying drawings in which:—

Fig. 1 is a sectional view of the cereal grain moisture measuring apparatus of the electrostatic capacity type comprising one embodiment of the invention; and

Figs. 2 and 3 show a control circuit of the embodiment shown in Fig. 1.

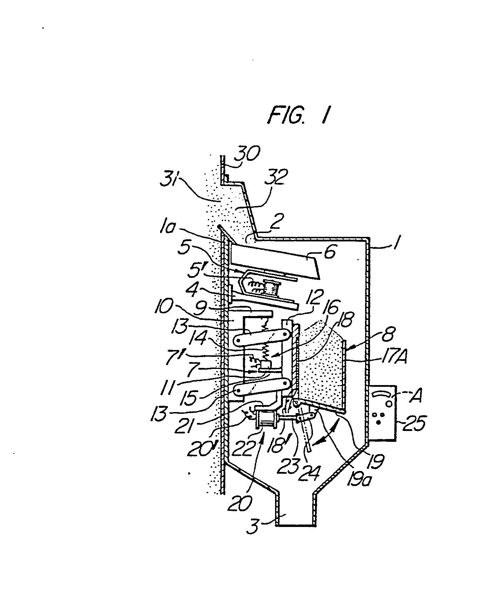

In Fig. 1, the numeral 1 designates a frame of one embodiment of the cereal grain moisture content measuring apparatus in conformity with the invention formed with a grain feeding port 2 on one side of its upper portion and with a grain discharging port 3 in its lower portion. The frame 1 includes one side wall 1a having attached to an upper portion of its inner surface a fixed plate 4 mounting thereon a grain feeding device comprising a grain feeding trough 6 provided with vibratory means 5. A weighing device 7 is supported on the inner surface of the side wall 1a beneath the fixed plate 4, and a vessel 8 for containing cereal grain to be measured for its moisture content is connected to one side of the weighing device 7.

The weighing device 7 comprises a support frame 10 affixed to the inner surface of the side wall 1a of the frame 1 and having an inwardly projecting suspension bar 9 formed at its upper end, a movable frame 12 located parallel to the support frame 10 as viewed vertically and having a cross bar 11 attached to its intermediate portion and projecting horizontally toward the support frame 10, and a plurality of connecting bars 13 connecting the movable frame 12 to the support frame 10 to enable the movable frame 12 to move vertically while being maintained in vertically parallel relation to the support frame 10. The suspension bar 9 is connected to the cross bar 11 by a spring scale 16 comprising a spring 14 and a weight detecting section 15.

Attached to a surface of the movable frame 12 opposite a surface thereof to which the cross bar 11 is attached is a cylindrical body 17A of the vessel 8 which is open at its top and has an electrostatic capacity sensor 18 mounted on its inner wall surface and an ON-OFF valve 19 pivotally mounted at its bottom. The ON-OFF valve 19 is adapted to be opened and closed by ON-OFF valve actuating means 20 comprising a bracket 21 attached to the lower portion of the movable frame 12, a solenoid 22 mounted on the bracket 21 and having a rod 23, and a link 24 pivotally connected at one end to the rod 23 and pivotally connected at the other end to a projection 19a on the undersurface of the ON-OFF valve 19.

The weighing device 7, vibratory means 5 and electrostatic capacity sensor 18 have electrical leads 7', 5' and 18' respectively, which are operatively connected, together with electric leads 20' of the ON-OFF valve actuating device 20, to a control circuit A mounted on an outer surface of the frame 1. The numeral 25 designates an indicator mounted on a casing of the control circuit A for indicating the measured moisture content of the cereal grains in the vessel 8.

In this embodiment, the frame 1 of the cereal grain moisture content measuring apparatus is attached to a grain tank 30 as of a grain drying apparatus, and the grain feeding port 2 of the frame 1 communicates with a grain sampling port 31 formed in the wall of the grain tank 30 through a grain flow passage 32. Thus a sample of the cereal grains in the grain tank 30 can be fed into the vessel 8 by flowing downwardly through the grain flow passage 32 and the grain feeding trough 6 described hereinabove.

In this embodiment, the spring scale 16 constitutes the weighing device 7. It is to be understood that the invention is not limited to this specific form of weighing device and that any other known weighing device, such as a weighted balance, or load cells, may be used.

Operation of the embodiment of the aforesaid construction will now be described. The sample of cereal grains discharged from the grain tank 30 through the grain sampling port 31 and flowing through the grain flow passage 32 on to the grain feeding trough 6 is fed into the vessel 8 from the lower end of the trough 6 by the action of the vibratory means 5. The cylindrical body 17A of the vessel 8 is constructed to have a predetermined volume. Thus, as the sample of cereal grains is fed into the vessel 8 in an amount commensurate with the predetermined volume of the cylindrical body 17A, the stack of the sample of grains forms an angle of repose at the top and excess grains overflow the side wall of the cylindrical body 17A so that the sample of cereal grains contained in the vessel 8 may not exceed a predetermined volume at all times. At this time, the cereal grain feeding operation is interrupted by rendering the vibratory means 5 inoperative as subsequently to be described, and the weighing device 7 is actuated to measure the weight of the sample of cereal grains of the predetermined volume. At the same time, the moisture content of the sample of cereal grains of the predetermined volume is sensed by the sensor 18, and the value of the moisture content determined by the sensor 18 is corrected in terms of predetermined density based on the weight of the sample in the control circuit A. The corrected value of the moisture content of the sample is indicated by the indicator 25. Then, the ON-OFF valve 19 is brought to an open position to allow the sample of cereal grains in the vessel 8 to be discharged therefrom. The aforesaid moisture content measuring operation is repeatedly carried out in cycles with a series of samples.

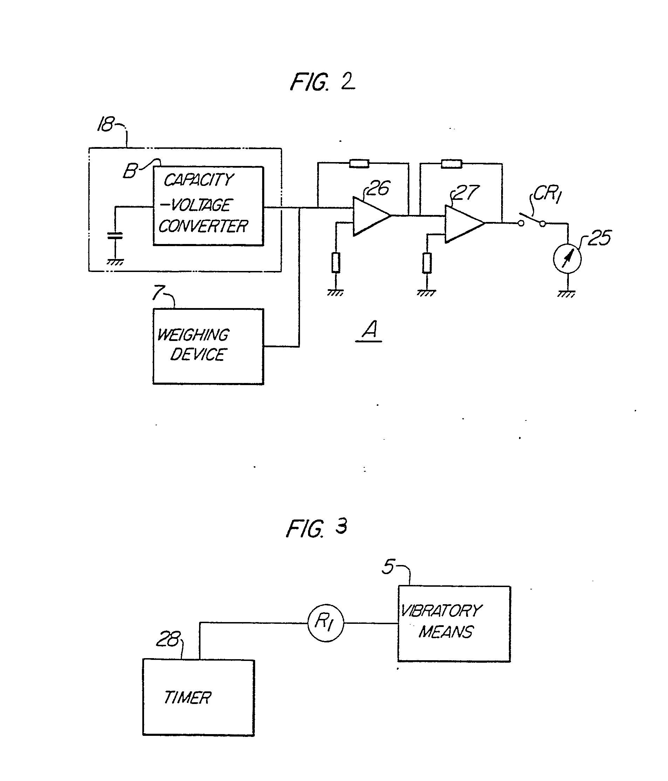

Figs. 2 and 3 show an example of the control circuit A used in the aforesaid moisture content measuring operation. In Fig. 2, outputs of the sensor 18 comprising a power source and a C-V converter (capacity-voltage converter) B and the weighing device 7 are connected to an input of a correction circuit 26 which is connected at its output to the indicator 25 via an amplifier 27 and a relay contact $CR_1$. A relay $R_1$ may be interposed between a timer 28 and the vibratory means 5 for the grain feeding trough 6, as shown in Fig. 3. The timer 28 energizes the relay $R_1$ when the predetermined volume of the sample of cereal grains is fed into the vessel 8 and an angle of repose is formed on the stack of the sample following lapse of a predetermined grain sample feeding time as shown in Fig. 1. The relay $R_1$ opens an ON-OFF contact (not shown) mounted on the vibratory means 5 to interrupt vibration of the grain feeding trough 6, to stop the feeding of the grain sample into the vessel 8. At the same time, the relay contact CR₁ shown in Fig. 2 is closed to allow a current to be passed from the correction circuit 26 to the indicator 25. Thus the weighing device 7 measures the weight of the vessel 8 containing the predetermined volume of sample of cereal grains and supplies a signal to the correction circuit 26 of the control circuit A; and at the same time the sensor 18 stably senses the electrostatic capacity of the cereal grains in the vessel 8 and supplies a signal to the correction circuit 26. In the correction circuit 26, the signals from the sensor 18 and weighing device 7 are compared with each other and the measured moisture content is corrected in terms of a predetermined density based on a change in the measured weight of the sample grains by suitably increasing or decreasing the moisture content value. The corrected value of moisture content obtained in this way is amplified by the amplifier 27 and transmitted to the indicator 25 to be indicated thereby. Following completion of the moisture content measuring operation, the solenoid 22 of the actuating means 20 of the ON-OFF valve 19 is energized by a signal from the timer 28 to open the ON-OFF valve 19, to thereby permit the grain sample in the vessel 8 to be discharged therefrom. The ON-OFF valve 19 is closed when all the grain sample is discharged from the vessel 8, to allow the next following moisture content measuring operation to be carried out.

From the foregoing description, it will be appreciated that in the cereal grain moisture content measuring apparatus according to the invention, a vessel having a sensor on its inner wall surface and formed with a discharge valve at its bottom is connected to a weighing device; a cereal grain feeding device is mounted above the vessel; and electric circuits of the weighing device and cereal grain grain feeding device are operatively connected to an electric circuit of the sensor. A sample of cereal grains flowing downwardly from the cereal grain feeding device is supplied to the vessel, and the weight of the sample of cereal grains in the vessel is measured and the moisture content thereof is sensed by the sensor after the cereal grain feeding is interrupted. The sensed moisture content is corrected in terms of a predetermined density based on a change in weight, so that an accurate moisture content of the sample of cereal grains can be determined. The disadvantage that the sensed moisture contents have often been erroneous due to variations in the density of grains can be eliminated, and the accurate value can be readily indicated. Moreover, the apparatus is of simple construction and free from mechanical and electrical failures and has improved performance. Thus the apparatus according to the invention is of great use in enabling cereal grains of high quality having a predetermined moisture content to be obtained on a substantially continuous basis in a smooth operation.

## Claims

1. Apparatus for measuring moisture content of cereal grains, comprising a vessel (8) for receiving successive samples of cereal grains each having a predetermined volume, said vessel (8) having a substantially tubular body (17A) with an upper open end and a lower open end, feeding means for feeding cereal grains into said vessel (8), sensor means (18) associated with said vessel (8) for detecting electrostatic capacity of a sample of cereal grains in said vessel (8) to generate a signal representative of the moisture content of the cereal grains, a measuring unit (7) for measuring the weight of each sample of cereal grains having said predetermined volume to generate a signal representative of the measured weight, and a correction circuit (26) electrically connected to said sensor means (18) and said measuring unit (7) for receiving the signal representative of the moisture content and the signal representative of the weight, and operating to correct the moisture content signal in accordance with changes in density of the cereal grains, characterized

in that said vessel (8) has associated therewith valve means (19) which is movable between a closed position in which it closes the lower open end of said vessel (8), to retain a sample of cereal grains to be measured in said vessel (8) and an open position in which it opens the lower open end of said vessel (8) to allow the measured cereal grain sample to be discharged out of said vessel (8), with actuating means (20) for moving said valve means (19) between the closed and open positions;

in that said feeding means comprises a feeding trough (6) having an upstream end, and a downstream end located above the upper open end of said vessel (8), and vibrating means (5) for vibrating said feeding trough (6), when said vibrating means (5) is activated and said valve means (19) is in its closed position, to cause cereal grains to be gradually fed into said vessel (8) by the feeding trough (6) until the thus fed cereal grains are overflowed from the upper open end of said vessel (8) to form an angle of repose at the top of the fed cereal grains so that a sample of the cereal grains having the predetermined volume is received in said vessel (8), said feeding trough (6) remaining stationary so that no cereal grains are fed when the vibrating means (5) is deactivated; and

in that signal-generating means (28) is electrically connected to said actuating means (20) for said valve means (19) and to said vibrating means (5) of said feeding means for generating and supplying a first signal to said vibrating means (5) to deactivate the same when a sample of the cereal grains having the predetermined volume has been fed into said vessel (8) by said feeding trough (6), and for generating and supplying a second signal to said actuating means (20) so as to cause the valve means (19) to be moved into said open position when the detection by said sensor means (18) and the measurement by said measuring unit (7) have been completed.

2. Apparatus as defined in Claim 1, characterized in that said sensor means (18) is mounted on an inner surface of said tubular body (17A) of said vessel (8) so as to be substantially flush with the inner surface of said tubular body (17A).

3. Apparatus as defined in Claim 1 or 2, characterized in that said signal-generating means comprises a timer (28) for setting a first time duration and generating said first signal after the lapse of the first time duration during which the cereal grains are fed by said feeding trough (6) into said vessel (8), and setting a second time duration and generating said second signal after the lapse of the second time duration during which the detection by said sensor means (18) and the measurement by said measuring unit (7) are conducted.

4. Apparatus as defined in any one of the preceding Claims, characterized in that the upstream end of said feeding trough (6) is arranged to receive cereal grains from within a grain tank (30), a sample of cereal grains from said tank (30) being fed into said vessel (8) upon activation of said vibrating means (5).

**Revendications**

1. Appareil pour la mesure de l'état hygrométrique de grains de céréales, comprenant une chambre (8) qui est alimentée successivement par des échantillons de grains de céréales ayant chacun un volume prédéterminé, la dite chambre (8) ayant un corps (17A) essentiellement tubulaire avec un bout supérieur ouvert et un bout inférieur ouvert, des moyens d'alimentation pour alimenter la dite chambre en grains de céréales, des moyens à sondes (18) disposés sur cette chambre pour détecter la capacité électrostatique d'un échantillon de grains de céréales dans la dite chambre afin de produire un signal représentant l'humidité dans les dits grains, un dispositif de pesage (7) pour mesurer le poids de chaque échantillon de grains de céréales ayant le dit volume, pour produire un signal représentant le poids mesuré, et un circuit de correction (26) raccordé électriquement aux dits moyens à sonde (18) et au dit dispositif de pesage (7), pour recevoir le signal représentant l'état hygrométrique et le signal représentant le poids et agissant de manière à corriger le signal représentant l'humidité en fonction des changements de densité;

cet appareil étant caractérisé en ce que ladite chambre (8) comprend des moyens à clapet (19) qui en position fermée ferment le bout inférieur de la chambre (8) pour y retenir un échantillon de grains de céréales à mesurer dans cette chambre (8) et qui en position ouverte ouvrent le bout inférieur de cette chambre (8) pour permettre à l'échantillon de grains de céréales de quitter la chambre (8), ainsi que des moyens de commande (20) pour mouvoir les moyens à clapet (19) entre leur position fermée et leur position ouverte;

en ce que les moyens d'alimentation comprennent un canal d'alimentation (6) ayant un bout supérieur et un bout inférieur situé au-dessus du bout supérieur de la dite chambre (8), et des moyens de vibration (5) faisant vibrer le dit canal d'alimentation (6) et qui — quand les dits moyens de vibration (5) sont mis en action et que les moyens à clapet (19) sont dans leur position fermée — provoquent l'alimentation graduelle de la dite chambre (8) en grains de céréales par le canal d'alimentation (6) jusqu'à ce que ces grains de céréales débordent du bout supérieur de la chambre (6) pour former un angle naturel de repos au sommet de ces grains de manière qu'un échantillon de grains de céréales ayant le volume prédéterminé soit accepté dans cette chambre, le canal d'alimentation (6) restant immobile de manière qu'aucun grains de céréales ne soit transporté pendant que les moyens de vibration sont au repos; et

en ce que des moyens produisant des signaux (28) sont raccordés électriquement aux moyens de commande (20) pour les moyens à clapet (19) aux moyens de vibration (5), pour créer un premier signal et le transmettre aux moyens de vibration (5) afin de mettre au repos ce dernier quand un échantillon de grains de céréales ayant le volume prédéterminé à été transporté dans la chambre (8) par le dit canal d'alimentation (6), et pour créer un deuxième signal et la transmettre aux moyens de commande (20) pour les moyens à clapet (19) afin de mouvoir ces derniers dans leur position ouverte quand la détection par les moyens à sonde (18) et la mesure par l'unité de mesure (7) sont terminées.

2. Appareil selon la revendication 1, caractérisé en ce que les moyens à sonde (18) montées sur une paroi intérieure du tube (17A) de la chambre (8) afin d'être aligné avec la surface intérieure du corps tubulaire (17A).

3. Appareil selon les revendications 1 ou 2, caractérisé en ce que les moyens produisant les signaux comprennent un timer (28) pour fixer une première période et pour créer le premier signal après cette première période pendant laquelle les grains de céréales sont transportés par le canal d'alimentation (6) dans la chambre (8), ainsi que pour fixer une deuxième période et produire le deuxième signal après la deuxième période pendant laquelle la détection par les moyens à sonde (18) et le dispositif de pesage (7) sont exécutées.

4. Appareil selon une des revendications 1 à 3, caractérisé en ce que le bout supérieure du canal d'alimentation (6) est disposé de manière à recevoir les grains de céréales d'un réservoir à grains (30), un échantillon de grains de céréales du réservoir (30) étant transporté dans la chambre (8) dès que les moyens de vibration (5) sont mis en action.

**Patentansprüche**

1. Vorrichtung zum Messen des Feuchtigkeitsgehaltes von Getreidekörnern, mit einem Behälter (8) zur Aufnahme von aufeinanderfolgenden, je ein vorbestimmtes Volumen aufweisenden Getreidekörnerproben, wobei dieser Behälter (8) einen im wesentlichen rohrförmigen Körper (17A) mit einem oberen und einem unteren offenen Ende aufweist, mit Zufuhrmitteln zur Zufuhr von Getreidekörnern in diesen Behälter (8), mit diesem Behälter (8) zugeordneten Sensormitteln (18) zur Festellung der elektrostatischen Kapazität einer in diesem Behälter (8) sich befindenden Getreidekörnerprobe zur Erzeugung eines dem Feuchtigkeitsgehalt der Getreidekörner entsprechenden Signals, mit einer Messeinheit (7) zum Messen des Gewichtes jeder der das vorbestimmte Volumen aufweisenden Getreidekörnerproben um ein dem gemessenen Gewicht entsprechendes Signal abzugeben, und mit einer elektrisch mit den Sensormitteln (18) und der Messeinheit (17) verbundenen Korrekturschaltungsanordnung (26) zur Verarbeitung des dem Feuchtigkeitsgehalt entsprechenden Signals und des dem Gewicht entsprechenden Signals zur Korrektur des Feuchtigkeitsgehaltsignals in Übereinstimmung mit Veränderungen in der Dichte der Getreidekörner, dadurch gekennzeichnet, dass dem Behälter (8) ein Verschluss (19) zugeordnet ist, welcher zwischen einer geschlossenen Stellung in welcher er das untere offene Ende des Behälters (8) verschliesst um eine zu messende Getreidekörnerprobe in diesem Behälter (8) zurückzuhalten, und einer offenen Stellung in welcher er das untere offene Ende des Behälters öffnet, um die gemessene Getreidekörnerprobe aus diesem Behälter (8) austreten zu lassen, bewegbar, mit Betätigungsmittel (20) zur Bewegung des Verschlusses (19) zwischen der geschlossenen und der offenen Stellung verbunden ist;

dass die Zufuhrmittel ein Zufuhrrinne (6) mit einem stromaufwärts sich befindenen Ende und einem oberhalb dem oberen offenen ende des Behälters (8) ageordneten, stromabwärts sich befindenden Ende, und Vibrationsmittel (5) zur Vibrierung der Zufuhrrinne (6) eingeschalteten Vibrationsmitteln (5) und in Schliessstellung sich befindendem Verschluss (19), aufweisen, um zu bewirken, dass die Getreidekörner über die Zufuhrrinne (6) allmählich in den Behälter (8) zugeführt werden, bis die derart zugeführten Getreidekörner am oberen offenen Ende des Behälters (8) überfliessen und sich auf der Oberseite der zugeführten Getreidekörner ein Schüttwinkel bildet, so dass in diesem Behälter (8) eine ein vorbestimmtes Volumen aufweisende Getreidekörnerprobe erhalten wird, und die Zufuhrrinne (6) bei ausgeschalteten Vibrationsmitteln (5) stationär bleibt, so dass keine Getreidekörner zugeführt werden; und

dass Signalerzeugungsmittel (28) elektrisch mit den Betätigungsmittel (20) des Verschlusses (19) und mit den Vibrationsmitteln (5) der Zufuhrmittel zur Erzeugung und Abgabe eines ersten Signals an die Vibrationsmittel (5) zur Deaktivierung derselben bei Erreichen des vorbestimmten Volumens der über die Zufuhrrinne (6) in den Behälter (8) eingefüllten Getreidekörnerprobe, und zur Erzeugung und Abgabe eines zweiten Signals an die Betätigungsmittel (20) zur Bewirkung einer Bewegung des Verschlusses (19) in die offene Stellung wenn die Abtastung mittels der Sensormittel (18) und die Messung mittels der Messeinheit (7) beendigt ist, verbunden sind.

2. Vorrichtung nach Anspruch 1, dadurch gekennzeichnet, dass die Sensormittel (18) auf einer nach innen gerichteten Oberfläche des rohrförmigen Körpers (17A) des Behälters (8) derart angeordnet, sind, dass sie sich praktisch in einer Ebene mit dieser nach innen gerichteten Oberfläche des rohrförmigen Körpers (17A) befinden.

3. Vorrichtung nach Anspruch 1 oder 2, dadurch gekennzeichnet, dass die Signalerzeugungsmittel ein Zeitschaltelement (28) zur Einstellung einer ersten Zeitdauer und Erzeugung des ersten Signals nach Ablauf der ersten Zeitdauer während welcher die Getreidekörner mittels der Zufuhrrinne (6) in den Behälter (8) eingeführt werden, und zur Einstellung einer zweiten Zeitdauer und Erzeugung des zweiten Signals nach Ablauf der zweiten Zeitdauer während welcher die Abtastung durch die Sensormittel (18) und die Messung mittels der Messeinheit (7) durchgeführt werden, aufweisen.

4. Vorrichtung nach einem der vorangehenden Ansprüche, dadurch gekennzeichnet, dass das stromaufwärts sich befindende ende der Zufuhrrinne (6) zur Aufnahme von innnerhalb einem Körnervorratsgefäss (30) sich befindenden Getreidekörnern angeordnet ist, und eine Probe an aus diesem Vorratsgefäss (30) stammenden Getreidekörnern nach Aktivierung der Vibrationsmittel (5) in den Behälter (8) zugeführt wird.

FIG. 1

*FIG.* 2

*FIG.* 3